# EUROPEAN PATENT SPECIFICATION

(11) **EP 2 091 439 B1**
(45) Date of publication and mention of the grant of the patent: **24.10.2012**
(21) Application number: 07864246.9
(22) Date of filing: 09.11.2007
(51) Int. Cl.: A61B 8/12, A61B 17/34, A61B 8/06, A61B 17/22, A61B 19/00

(54) **DEVICES FOR CREATING PASSAGES AND SENSING FOR BLOOD VESSELS**
VORRICHTUNGEN FÜR DIE ERZEUGUNG VON DURCHGÄNGEN UND MESSUNGEN FÜR BLUTGEFÄSSE
DISPOSITIFS POUR CREER DES PASSAGES ET DETECTER DES VAISSEAUX SANGUINS

(30) Priority: 22.11.2006 US 867076 P
(43) Date of publication of application: 26.08.2009
(73) Proprietor: Broncus Technologies, Inc., Mountain View, CA 94043 (US)
(72) Inventor: GWERDER, Eric, Fremont, California 94536 (US); KEAST, Thomas M., Sunnyvale, California 94089 (US); ROSCHAK, Edmund J., Mission Viejo, California 92692 (US)
(74) Representative: Price, Nigel John King
(86) International application number: PCT/US2007/084330
(87) International publication number: WO 2008/063935

(56) References cited:
- WO-A2-2005/006963
- US-A1- 2002 111 620
- US-A1- 2003 204 138
- US-A1- 2004 158 143
- US-A1- 2005 056 292
- US-B1- 6 306 097

## Description

### FIELD OF THE INVENTION

The invention is directed to devices for sensing movement within tissue, and then creating passages in the tissue. In one variation, sensing movement may comprise sensing for the presence or absence of blood vessels. Variations may include further detecting for these blood vessels in the passages created by the device.

### BACKGROUND OF THE INVENTION

It was found that creation of collateral channels in COPD patients allowed expired air to pass out of the lungs and decompressed hyper-inflated lungs. Such methods and devices for creating and maintaining collateral channels are discussed in U.S. Patent No. 6,692,494; U.S. Patent Application Nos. 09/947,144, 09/946,706, and 09/947,126 all filed on September 4, 2001; US Patent Application No. filed on September 4, 2002; US Patent Application No. 11/335,263, filed on January 18, 2006; Attorney Docket number BRON-N-E004.05-US, US Patent Application No. (TBD) and filed on November 22, 2006.

The creation of these channels also seems to overcome the shortcomings associated with bronchodilator drugs and lung volume reduction surgery. Placement of an implant within the channel further increased the duration of the treatment.

However, because creation of the opening/channel is typically performed within the airway under bronchoscopic observation, care must be taken so as not to rupture a pulmonary vessel that lies beneath or outside of the airway wall. The need to avoid rupturing vessels that may be hidden by the airway walls is also evident when a surgeon attempts to obtain a biopsy sample from within the bronchial tree. In addition, because the location of the pulmonary vessels varies between patients, care must also be taken when working within the channel or biopsy site. For instance, although a channel may be created without puncturing a blood vessel, the subsequent dilation, insertion of an implant, and/or removal of biopsy material may perforate vessels that were otherwise undetected during the creation of the channel.

The problem is compounded when accounting for the tidal motion of lungs. For example, because the target site moves due to the tidal motion of the lungs (as a result of the mechanics of breathing), it is difficult to visually identify an area that was previously scanned unless the scanning device remains relatively stationary against the tissue. Moreover, the difficulty increases when considering that the procedure takes place through the camera of a bronchoscope or endoscope.

In view of the above, a need remains to increase the safety when creating openings in tissue so as not to rupture a blood vessel. Such a device may have applications outside of the lung in any situation where there is a need to locate blood vessels or other fluid carrying vessels prior or during creation of an opening in the tissue.

US 2003/204138 A1 discloses a guiding catheter including an inner guide moveably disposed within an outer guide. Both inner and outer guides include inner and outer balloons, respectively, located at the distal tip of the guides. The inner guide balloon is fluted, thereby allowing blood to flow past the implanted balloon when inflated. The outer balloon is substantially annular in shape and is deployable as an occlusion balloon. One or more sensors is provided at a distal end of the guiding catheter to assist in locating a destination vessel or structure of interest. A sensor can be mounted to the outer guide, the inner guide, or both. Sensor arrangements can include one or more of an optical sensor, infrared sensor, ultrasound sensor, pressure sensor, temperature sensor, flow sensor and oxygen sensor.

### SUMMARY OF THE INVENTION

According to the present invention there is provided the medical device of claim 1.

Preferred aspects of the medical device arc set out in the dependent claims.

The invention relates to creation of passages and/or removal of tissue while allowing sensing of blood vessels that may be in or around the area of the passage. Although specific reference is made to use of the subject invention within the lungs, it is noted that the invention may also be used within various other parts of the body that have a need for such safety measures.

The device allows for sensing blood vessels in tissue, and allows for creation of a passage or opening without moving the sensing element.

The dilation member is typically used to dilate the opening created by the device. As such it may be a non-distensible balloon.

The sensing assembly is used to scan the tissue to minimize causing undesirable injury to the patient. As discussed below, any number of sensing modes may be incorporated into the device. However, it was found that Doppler ultrasound transducer assemblies perform acceptably when sensing for blood vessels within tissue. In certain variations, the sensing assembly may be configured to puncture the tissue and create the opening. However, in other variations, the sensing assembly will have a blunted tip to minimize undesirable tissue damage.

The device may be used in methods of treating tissue, where the method includes selecting an,area in the tissue for treatment, advancing a device into the lung to a tissue site, scanning the tissue site with the sensing assembly for the presence or absence of blood vessels, creating an opening with the device without removing the sensing assembly from the tissue site, and dilating the opening with the dilation assembly.

The methods may include treating tissue to assist in decompressing hyper inflated lung tissue. Alternatively, the methods may include scanning of tissue during a biopsy or other medical procedures where perforating a blood vessel could result in injury to a patient.

The selecting step may be performed with direct visual imaging from the scope type device and/or may be performed with various types of non-invasive imaging equipment such as: x-ray, acoustic imaging, MRI. PET, computed tomography (CT) scans or other such imaging.

The step of creating the opening with the device may include using the dilation assembly or the sensing assembly to create an opening at the treatment site or adjacent to the treatment site (but within an acceptable range so that the scanning covers the tissue being penetrated).

In certain variations, the sensing assembly may also be inserted into the opening (prior to or after dilation) to ensure that a blood vessel or other organ was not missed when scanning the surface of the tissue.

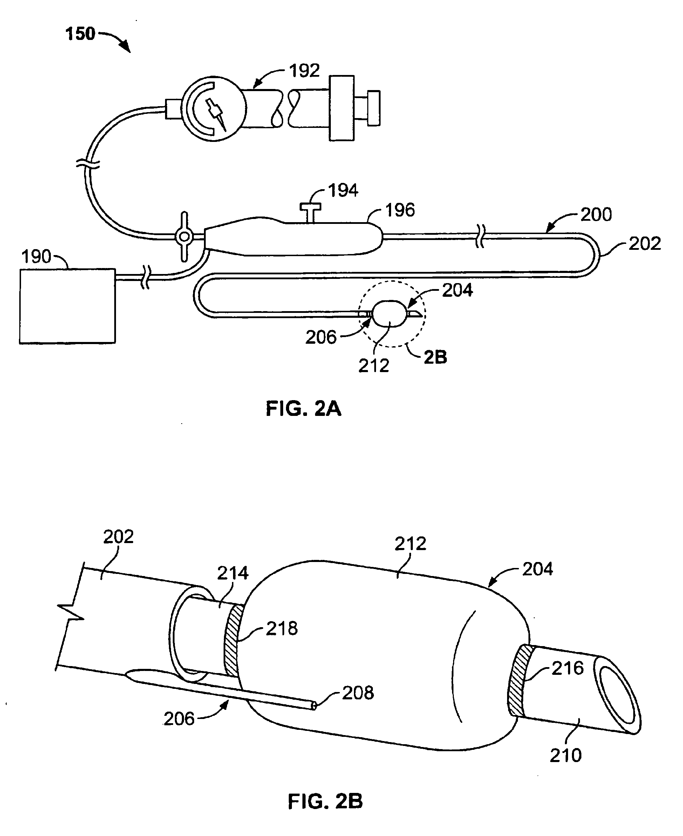

Embodiments of medical device in accordance with the present invention will now be described, by way of example only, with reference to the accompanying drawings. It should be noted that the medical devices illustrated in Figs. 2A, 2B, 4A and 7A-7E are not in accordance with the present invention in the precise form illustrated.

### BRIEF DESCRIPTION OF THE DRAWINGS

Figs. 1A-1C illustrate various states of the natural airways and the blood-gas interface.

Fig. 1D illustrates a schematic of a lung demonstrating a principle of the effect of collateral channels placed therein.

Fig. 2A shows a system as described herein.

Fig. 2B shows a far end of a catheter member with a dilation member in an actuated state.

Fig. 3A and 4B shows a variation of the end of the device.

Figs. 4A and 4B show various cross sectional views of devices and the expandable member being attached to a dilation member.

Figs. 5A-5B illustrate a non-exhaustive sample of variations of transducer assemblies.

Figs. 6A-6D, illustrate possible variations of optional tips for use with the transducer assembly.

Figs. 7A-7D illustrates examples of using the device to scan, create and dilate an opening in tissue.

Fig. 7E illustrates an optional step of scanning the opening before or after dilation of the opening.

### DETAILED DESCRIPTION OF THE INVENTION

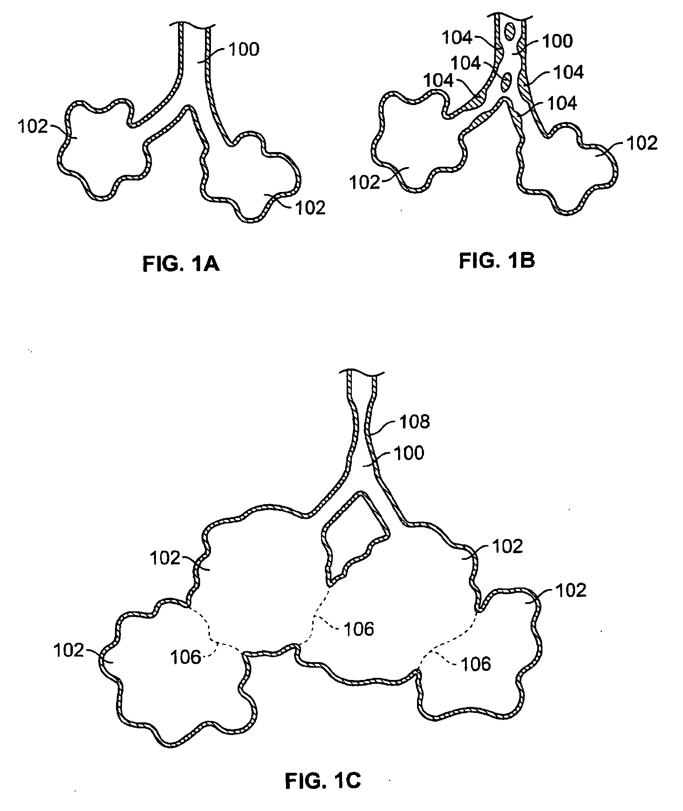

Fig. 1A shows a simplified illustration of a natural airway **100** which eventually branches to a blood gas interface **102**. Fig. 1B illustrates an airway **100** and blood gas interface **102** in an individual having COPD. The obstructions **104** (e.g., excessive mucus resulting from COPD, see above) impair the passage of gas between the airways **100** and the interface **102**. Fig. 1C illustrates a portion of an emphysematous lung where the blood gas interface **102** expands due to the loss of the interface walls **106** which have deteriorated due to a bio-chemical breakdown of the walls **106**. Also depicted is a constriction **108** of the airway **100**. It is generally understood that there is usually a combination of the phenomena depicted in Figs. 1A-1C. More usually, the states of the lung depicted in Figs. 1B and 1C are often found in the same lung.

As will be explained in greater detail below, the production and maintenance of collateral openings or channels through airway walls permits expired air to pass directly out of the lung tissue and into the airways to ultimately facilitate exchange of oxygen into the blood and/or decompress hyper inflated lungs. The term 'lung tissue' is intended to include the tissue involved with gas exchange, including but not limited to, gas exchange membranes, alveolar walls, parenchyma, airway walls and/or other such tissue. To accomplish the exchange of oxygen, the collateral channels allow fluid communication between an airway and lung tissue. Therefore, gaseous flow is improved within the lung by altering or redirecting the gaseous flow within the lung, or entirely within the lung.

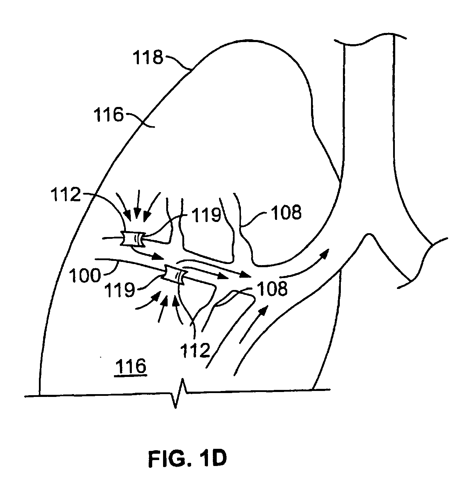

Fig. 1D illustrates a schematic of a lung **118** to demonstrate a benefit of the production and maintenance of collateral openings or channels through airway walls. As shown, a collateral channel **112** (located in an airway wall **110**) places lung tissue **116** in fluid communication with airways **100** allowing expired air to directly pass out of the airways **100**. The term channel is intended to include an opening, cut, slit, tear, puncture, or any other conceivable artificially created opening. As shown, constricted airways **108** may ordinarily prevent air from exiting the lung tissue **116**. In the example illustrated in Fig. 1D, there is no implanted structure placed in the collateral channel **112**. However, conduits or implants **119** may be placed in the collateral channels **112** to assist in maintaining the patency of the collateral channels **112**. Examples of conduits may be found in the applications discussed above. While there is no limit to the number of collateral channels which may be created, it is preferable that 1 or 2 channels are placed per lobe of the lung. For example, the preferred number of channels is 2-12 channels per individual patient. In current trials, it was found that 1-4 channels placed per lobe of the lung and 4-16 channels per individual patient was preferable. This number may vary on a case by case basis. For instance, in some cases an emphysematous lung may require 3 or more collateral channels in one or more lobes of the lung.

The present invention includes a device which is able to detect the presence or absence of a blood vessel by placing a front portion of the device in contact with tissue and then create an opening in the tissue without having to remove the device from the tissue.

Fig. 2A illustrates a sectional side view of a system **150**. The system generally includes a device **200** for sensing the presence of blood vessels and creating passages in tissue. The device **200** includes a catheter member **202** and dilation member **204** extending through the catheter member **202**. In the system shown, the dilation member **204** is shown in an actuated position outside of the catheter member **202**. However, as discussed herein, the dilation member **204** is moveable relative to the catheter member **202** and sensing assembly **206** so that it can be withdrawn relative to the sensing assembly **206** and/or the catheter member **204**. In this system, the dilation member **204** is coupled to an actuator **194** that is slidably located on a handle **196** at a near end of the catheter **202**. However, in alternate systems, the dilation member may also extend from the near end of the catheter member **202**. In such a case the medical practitioner moves the dilation member **204** relative to the catheter member **202** to insert the dilation member **204** into the tissue.

Typically, the catheter member **202** is sufficiently flexible and has a length that allows for the far end of the catheter member **202** to reach target sites when the device **150** enters the body through a bronchoscope or endoscope. Some devices described herein can be constructed to be stiff and inflexible. However, for most procedures, the device has sufficient flexibility, column strength and length to access the tissue targeted for treatment within tortuous anatomy (such as those devices intended for use in small airways of the lung). Accordingly, for devices used to create collateral channels within lungs, the length of the device should preferably be between 0.46-1.52m (1.5-5 ft) long (preferably 1.22-1.52m (4-5 ft)) in order to reach the targeted airways.

The device **200** may be coupled to a control system **190** that is configured to assist the medical practitioner in detecting whether blood vessels are at or near a particular target site. The system **150** also includes a fluid source **192** for dilation of the tissue after the device creates the openings. The fluid source may be any standard device used to pressurize gas or liquid into an expandable dilation member **212** located at the far end of the device **200**. Although illustrated to be a syringe type device, the fluid source 192 may a compressor type device as well.

When used, the control system **190** is coupled to a sensing assembly **206** that extends from the far end of the catheter member **202**. The sensing assembly **206** and control system **190** may be any type of unit that confirms the presence or absence of blood vessels. As such, it may be a thermal based system, light based system, ultrasound based system, or Doppler based system. For exemplary purposes, the control system **190** and sensing assembly **206** are discussed herein as being a Doppler ultrasound system. As illustrated, the sensing assembly **206** includes a sensing tip **208** that is coupled to the power supply **190** by conducting members (e.g., wires) extending along the catheter member **202** (either internally or externally to the catheter member 202). In any case, these conducting members provide the energy and controls for the sensing assembly **206**. In the case of Doppler ultrasound, the conducting members couple an ultrasound source **190** to the sensing tip **208** that comprises an ultrasound transducer assembly or lens.

Variations of the device may include conducting members that comprise a series of wires, with one set of wires being coupled to respective poles of the transducer, and any number of additional sets of wires extending through the device. In addition, the sensing assembly **206** may have more than one sensing surface disposed along the portion of the sensing assembly **206** that extend from the device.

As discussed herein, any conventional sensing type probe may be used to detect the blood vessel. When using Doppler ultrasound to detect the presence of blood vessels within tissue, the ultrasound can operate at any frequency in the ultrasound range but preferably between 2Mhz-30Mhz. It is generally known that higher frequencies provide better resolution while lower frequencies offer better penetration of tissue. In the present invention, because location of blood vessels does not require actual imaging, there may be a balance obtained between the need for resolution and for penetration of tissue. Accordingly, an intermediate frequency may be used (e.g., around 8Mhz). A variation of the invention may include inserting a fluid or gel into the airway to provide a medium for the Doppler sensors to couple to the tissue to detect blood vessels. In those cases where fluid is not inserted, the device may use mucus found within the airway to directly couple the sensor to the wall of the airway.

As noted above, Doppler ultrasound was found to be an efficient way to identify blood vessels. As such, the control system **190** can be configured to communicate with an analyzing device or control unit adapted to recognize the reflected signal or measure the Doppler shift between the signals. The source signal may be reflected by changes in density between tissues. In such a case, the reflected signal will have the same frequency as the transmitted signal. When the source signal is reflected from blood moving within a vessel, the reflected signal has a different frequency than that of the source signal. This Doppler Effect permits determination of the presence or absence of a blood vessel within tissue. The Doppler system described herein comprises a Doppler ultrasound mode of detection. However, additional variations include transducer assemblies that allows for the observation of the Doppler Effect via light or sound as well.

Regardless of the mode incorporated by the sensing assembly, the system **150** may include a user interface that allows the user to determine the presence or absence of a blood vessel at the target site. Typically, the user interface provides an audible confirmation signal. However, the confirmation signal may be manifested in a variety of ways (e.g., light, graphically via a monitor/computer, etc.)

Although depicted as being external to the device, it is contemplated that the control system **190** may alternatively be incorporated into the device **200**. Moreover, the system **150** may incorporate any number of connectors or fitting that allow for either permanent or detachable connections of the fluid source, control system and/or any other auxiliary systems used with the system **150**.

Fig. 2B illustrates an expanded view of the dilation member **204** extending out of the far end of the catheter member **202**. As shown, the dilation member includes a distal tip **210** and an expandable member **212** located adjacent to the tip **210**. In the illustration shown, the tip **210** comprises a sharpened tip. When the tip **210** comprises such a tissue piercing configuration, the tip **210** is able to penetrate soft tissue or other composite type tissue (e.g., that of an airway wall). Such a configuration may include a stainless steel thin walled tubing such as a hypo-tube, cannula tubing such as that used for needles, solid sharpened mandrel, double beveled needle tip, etc. located at a far end of a shaft **214** of the dilation member **204**. The tip **210** described herein will be sharp or have a sufficiently small surface area such that insertion of the tip **210** through tissue occurs by advancement of the dilation member **210** (or a component thereof). It is contemplated that, where possible, any of the tissue piercing tips described herein may be incorporated into any of the variations described herein. Alternatively, the tip **210** may be rounded or blunted. Regardless, the tip **210** is configured to facilitate entry of the expandable member **212** into tissue for dilation of the tissue (whether the opening is created by the tip **210** or the sensing assembly **206**).

Fig. 2B also illustrates the dilation member **204** having an expandable member **212**. Although the expandable member **212** is shown as being a balloon, alternate expandable members (such as those that allow for mechanical dilation such as a basket, a dilator, etc.) are within the scope of the disclosure. In such a case, the fluid source **192** is naturally replaced with the appropriate actuation mechanism. The use of a balloon **212** allows for controlling pressure during dilation of the passage in tissue created by the tip **210**.

Variations of the device **200** can be designed for use in tough tissue that is resistant to radial expansion (such as an airway wall). In such variations, the balloon may comprise non-distensible balloon to overcome the toughness of the tissue. Non-distensible balloons are generally made up of relatively inelastic materials consisting of PET, nylons, polyurethanes, polyolefins, PVC, and other crosslinked polymers. Therefore, use of a non-distensible balloon allows for easier expansion of tissue because the non-distensible balloon permits high pressurization (> 6 atm). Moreover, non-distensible balloons generally inflate in a uniform shape (radially, longitudinally, or both) since the balloon unfolds to assume an expanded shape. In contrast, distensible balloons typically expand in shape when pressurized. In any case, it should be noted that distensible and/or non-distensible balloons may be used in the present invention depending upon the application.

Non-distensible balloons typically occupy a greater mass than distensible balloons because the non-distensible balloon is inelastic and is folded in an unexpanded shape. Therefore, variations of the invention include non-distensible balloons having a working diameter (or diameter in an unexpanded shape) that is close to the diameter of the tip **210** or shaft **214**. This allows insertion of the unexpanded balloon into the opening created by the piercing member. Accordingly, balloons of the present invention may include thin walled balloons, balloons with small distal profiles, balloons with distal ends that are close in actual diameter to the diameter of the piercing member, or balloons that folds into low profile state, or balloons having a combination of these features.

Fig. 2B also illustrates markers **216**, **218** placed on the dilation member **204**. In this variation, markers **216**, **218** are placed on the distal and proximal ends of the expandable member **204**. However, variations include use of a single marker. The markers **216**, **218** may be radiopaque, and/or visually apparent. In the latter case, a visually apparent marker permits the medical practitioner to confirm location of the expandable member **212** prior to dilation. The marker **218** located at the proximal end of the expandable member **212** assists the practitioner in determining when the expandable member **212** clears the catheter member **202**.

The markers **216**, **218** may be a ring of biocompatible polymer and may be selected to provide contrast so that it may be identified as the medical practitioner views the device through an endoscope or bronchoscope. For example, the bronchoscope will usually contain a light-source that illuminates the target area. Therefore, the markers may be fabricated to reflect or refract the light in a different manner from the remainder of the device. In one variation, the markers may be the same color as the remainder of the device, or partially transparent, or entirely transparent, but is identifiable because the markers reflect or refracts light differently than the remainder of the device.

The markers may be made using a number of techniques. In one example, the mark is a ring formed of silicone and is white. The polymeric ring may be spun onto the dilation member. In another example the marker is a ring formed of silicone and is black. In another example the mark is a ring formed by suspending gold particulates in a polymer allowing for visual and radiopaque contrast.

The shape of the marker is not limited to a thin ring. The visualization mark may be large. The markers may, for example, be a white coating disposed on the shaft of the dilation member. It should be noted that variations of the invention include coloring the balloon itself, or other expandable member, to provide contrast like the marker.

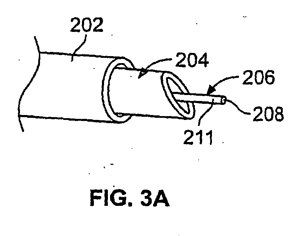

Fig. 3A illustrate, a sensing assembly 206 configuration for the far end of the catheter member **202** which renders the medical device in accordance with the present invention.

Fig. 3A shows a variation of the earlier described sensing assembly **206** having a segment **211** that extends from within the tip of the dilation member **204**. As discussed herein, in most variations, the sensing tip **208** is fixed and extends a distance beyond the catheter **202** so that the tip **208** may be pressed against tissue to scan for blood vessels or other structures. Once the practitioner locates an acceptable site, the practitioner advances the dilation member to create an opening with the distal tip **210**. Although placing the sensing assembly **208** through the dilation member **204** may offer more precise scanning of the tissue prior to creation of any openings, this construction may be more complicated as the added structure of the sensing assembly **204** may make it more difficult to navigate the catheter member **202** through tortuous anatomy.

It is noted that the variation of Fig. 3A may also comprise an offset segment **211** construction while remaining within the distal tip of the dilation member. In any case, the offset feature is useful when navigating the device **200** through tortuous anatomy. In one aspect, when the offset feature is outside of the dilation member, it eliminates the need for the segment **211** from extending through the length of the catheter member. All constructions of the offset feature also reduces the chance that the sensing tip **208** will be obscured by the catheter member **202** when viewed by the end of the bronchoscope or endoscope.

The degree to which the segment **211** and sensing tip **208** extend from the catheter member **202** may vary depending on the particular application. For example, in certain variations, the sensing tip may be immediately distal to the end of the catheter member. In alternate variations, the sensing tip may extend as shown in the drawings. Such a construction is useful when the practitioner desires to insert the sensing tip **208** into an opening within the tissue to perform additional scanning.

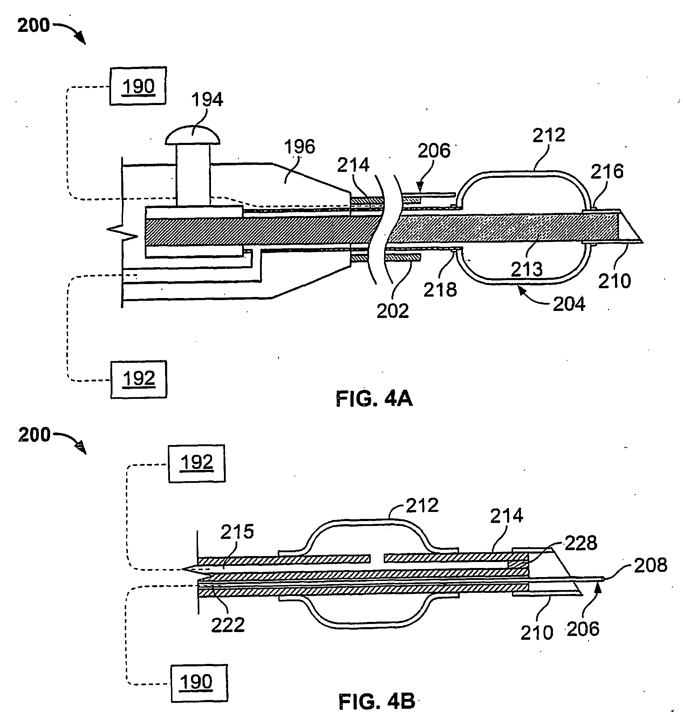

Figs. 4A and 4B illustrate various exemplary constructions of the device **200**. In all cases, for illustrative purposes, the expandable member **212** is shown as being in an expanded state. During insertion of the device into the body, the expandable member **212** can be reduced to fit within the catheter member **202** or to the same size as the catheter member.

Fig. 4A shows a cross sectional view of a device **200** as described herein where the dilation member **204** is actuated, which device is not in accordance with the present invention. In this device, the sensing assembly **206** is affixed to an exterior of the catheter member **202** and coupled to the control unit **190** via wires or other conducting members. As shown, the dilation member **204** is affixed to an actuator **194**. However, the dilation member **204** is reinforced with a support member **213** that extends from the distal tip **210** through to the handle portion **196**. To actuate the dilation member **204**, the operator advances the actuator **194** to push a polymeric shaft **214** and the support member **213**. As a result, the support member **213** carries the load and increases the amount of force transferred to the distal tip **210**. Alternatively a distal end of the support member **213** may be used without a distal tip **210**. In such a case, the distal end of the support member **213** may be sharpened or otherwise configured to be equivalent to the distal tip **210** of the dilation member **204**. This device also shows the distal end of the expandable member **212** being coupled to the distal tip **210** and the proximal end being coupled to the shaft **214**. While both ends of the balloon may be constructed to be the same size, the distal end of the balloon may be a smaller size to ease insertion of the tip **210** and distal end of the un-inflated balloon into the tissue.

The support member may be a flexible mandrel or tube. Alternatively, it may be a braided member that provides flexibility for navigation through tortuous anatomy and column strength for driving the end of the device into tisse.

The use of a reinforcing or support member **213** provides the device **200** with considerable flexibility to navigate through tortuous anatomy while maintaining greater column strength over a device having a non-reinforced polymer shaft. However, a reinforcing member may be incorporated into the shaft **214**. Alternatively, no reinforcing member may be used.

Fig. 4A, also illustrates the interior of the expandable member being coupled to the fluid source **192**. The illustration is intended for exemplary purposes as any number of couplings may be employed to fluidly couple the expandable member to the fluid source.

Fig. 4B illustrates a variation of a shaft **214** of a dilation member. In this example the catheter member is omitted. As shown, a first lumen **215** fluidly couples the fluid source **192** to the expandable member **212**. A second lumen **222** carries the sensing assembly **206**. In this variation, the sensing assembly **206** is affixed to a catheter member so that the dilation member moves over the sensing assembly **206** to penetrate tissue. As also shown, the first lumen **215** may be sealed with a plug **228** or other occlusion member to prevent fluid from escaping from the device.

The devices described above may be constructed from any standard medical grade material. For example, the shafts and catheters may comprise commercially available medical-grade flexible tubing. For example, the elongate member may comprise PTFE, polyimide, polypropylene, or other such engineered polymers such as Hytrel® manufactured by DuPont.

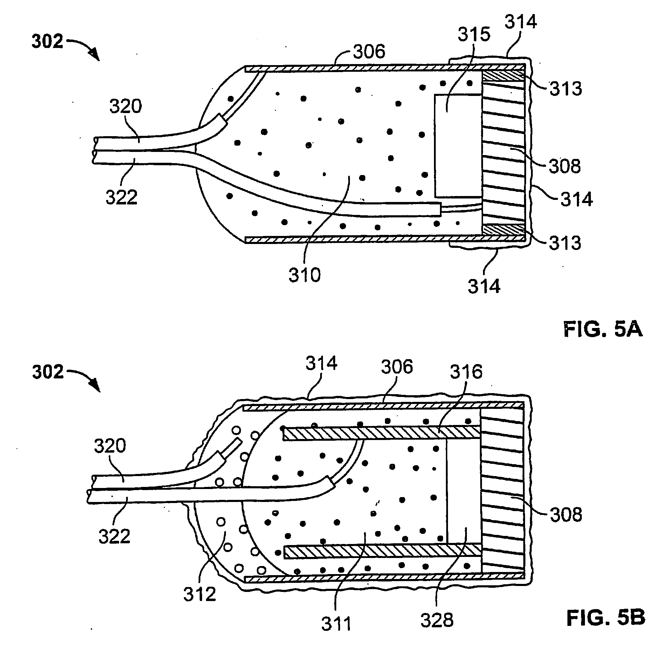

Figs. 5A-5B illustrate a non-exhaustive sample of variations of transducer assemblies **302** configured to reduce an overall size of the assembly. It is noted that the invention may use any type of transducer assembly. Fig. 5A illustrates a cross-sectional view of a basic variation of a transducer assembly **302**. The transducer assembly **302** includes at least one transducer **308** (e.g., a piezoelectric transducer.) In this variation, the front surface of the transducer **308** comprises a first pole and the rear surface comprises a second pole.

The transducer or transducers may comprise a piezo-ceramic crystal (e.g., a Motorola PZT 3203 HD ceramic). In the current invention, a single-crystal piezo (SCP) is preferred, but the invention does not exclude the use of other types of ferroelectric material such as poly-crystalline ceramic piezos, polymer piezos, or polymer composites. The substrate, typically made from piezoelectric single crystals (SCP) or ceramics such as PZT, PLZT, PMN, PMN-PT; also, the crystal may be a multi layer composite of a ceramic piezoelectric material. Piezoelectric polymers such as PVDF may also be used. Micromachined transducers, such as those constructed on the surface of a silicon wafer are also contemplated (e.g., such as those provided by Sensant of San Leandro, CA.) As described herein, the transducer or transducers used may be ceramic pieces coated with a conductive coating, such as gold. Other conductive coatings include sputtered metal, metals, or alloys, such as a member of the Platinum Group of the Periodic Table (Ru, Rh, Pd, Re, Os, Ir, and Pt) or gold. Titanium (Ti) is also especially suitable. The transducer may be further coated with a biocompatible layer such as Parylene or Parylene C.

The covering **306** of the transducer assembly **302** may contain at least a portion of the transducer **308**. In some variations of the invention, the covering **306** may comprise a conductive material. In such cases the covering **306** itself becomes part of the electrical path to the first pole of the transducer **308**. Use of a conductive covering **306** may require insulating material **313** between the sides of the transducer **308**, thereby permitting a first conductive medium **314** to electrically couple only one pole of the transducer **308** to the covering **306**.

At least a portion of the front surface of the transducer **308** will be in contact with the conductive medium **314**. The conductive medium **314** permits one of the poles of the transducer **308** to be placed in communication with a conducting member that is ultimately coupled to a power supply. As shown in this example, the conductive medium **314** places the pole of the transducer **308** in electrical communication with the covering **306**. In some variations the conductive medium **314** may coat the entire transducer **308** and covering **306**. Alternatively, the conductive medium **314** may be placed over an area small enough to allow for an electrical path between a conducting member and the respective pole of the transducer **308**. The conductive medium **314** may be any conductive material (e.g., gold, silver, tantalum, copper, chrome, or any biocompatible conductive material, etc. The material may be coated, deposited, plated, painted, wound, wrapped (e.g., a conductive foil), etc. onto the transducer assembly **302**.

The transducer assembly **302** depicted in Fig. 5A also illustrates conducting members **320**, **322** electrically coupled to respective poles of the transducer **308**. Optionally, the conducting members **320**, **322** may be encapsulated within an epoxy **311** located within the covering **306**. The epoxy **311** may extend to the transducer **308** thereby assisting in retaining both the conducting members **320**, **322** and transducer **308** within the covering. It may also be desirable to maintain a gap **328** between the transducer **308** and any other structure. It is believed that this gap **228** improves the ability of the transducer assembly **302** to generate a signal.

Fig. 5B illustrates another variation of a transducer assembly **302**. In this variation, the conductive medium **314** extends over the entire transducer covering **306**. Accordingly, the covering **306** may be made of a non-conducting material (e.g., a polyamide tube, polyetherimide, polycarbonate, etc.) The transducer assembly **302** may further comprise a second tube **316** within the covering **306**. This second tube **316** may be a hypo-tube and may optionally be used to electrically couple one of the conducting members to a pole of the transducer **308**. As shown, the covering **306** may contain a nonconductive epoxy **310** (e.g., Hysol 2039/3561 with Scotchlite glass microspheres B23/500) which secures both the conducting member and the second tube **316** within the covering 306. This construction may have the further effect of structurally securing the transducer 308 within the assembly **302**. Again, a gap **328** may or may not be adjacent to the transducer to permit displacement of the transducer **308**.

Fig. 5B also illustrates the assembly **302** as having a conductive epoxy **312** which encapsulates the alternate conducting member **320**. An example of a conductive epoxy is Bisphenol epoxy resin with silver particulates to enable conductivity. The particulates may be from 70-90% of the resin composition. The resin may then be combined with a hardener (e.g., 100 parts resin per 6 parts hardener.) The conductive epoxy **312** is in electrical communication with the conductive medium **314** allowing for a conductive path from the conducting member **320** to the conductive medium **314**. Accordingly, use of the conductive epoxy **312** secures the conducting member **320** to the assembly **302** while electrically coupling the conducting member **320** to the transducer via the conductive coating **314**.

Although variations of the transducer assembly include a tip and housing, the invention may omit the transducer covering and other structures not necessary to generate a source signal and receive a reflected signal. Therefore, it is contemplated that the invention may simply have a transducer that is coupled to a controller.

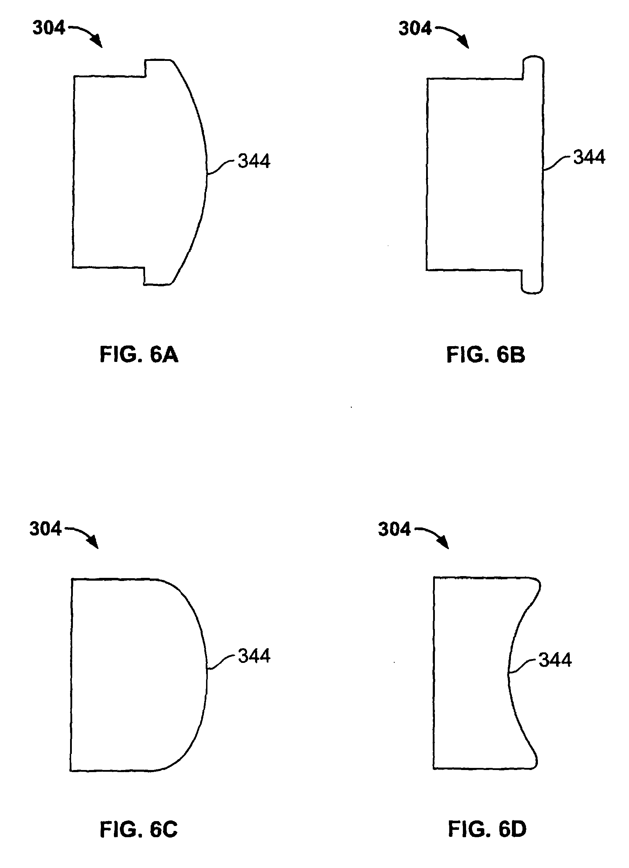

When used in the devices **200** described herein, the tip **208** of the sensing assembly may comprise the transducer **308** shown above, or the coating **314**. In alternative variations, the tip **208** of the sensing assembly may comprise a tip **304** that is affixed to the transducer assembly **302** and as shown in Figs. 6A-6D.

Figs. 6A-6D, illustrate possible variations of tips **304** for use with the transducer assembly. It is noted that these variations are provided for illustrative purposes and are not meant to be exhaustive. The tips **304** of the present invention may function simply as a blunting tip (but still passes and receives ultrasound signals) or as a lens to disperse and/or direct the signal over a substantial portion of the outer surface of the tip **304**. When configured to function as a lens, the tip **304** is adapted to disperse and/or direct (e.g., by diffraction) a reflected signal towards the transducer (not shown in Figs. 6A-6D). Accordingly, given the above described configuration, the inventive device **300** will be able to detect vessels with substantially most of the tip **304**. The tip may comprise a signal directing means.

When configured to function as a lens, the tip **304** is designed such that it interferes and redirects the signals in a desired direction in a manner like a lens. It also may be desirable to place an epoxy between the tip **304** and the transducer. Preferably, the epoxy is thin and applied without air gaps, bubbles or pockets. Also, the density/hardness of the epoxy should provide for transmission of the signal while minimizing any effect or change to the source signal. The configuration of the transducer assembly **302** permits the lens tip **304** to disperse a signal over a substantial portion of its outer surface **244**. The lens tip **304** also is adapted to refract a reflected signal towards the transducer **308**. Accordingly, given the above described configuration, the inventive device will be able to detect vessels with any part or substantially the entire lens tip **304** that contacts tissue.

Although the tip of the present invention is able to transmit a source signal and receive a reflected signal, the invention is not limited to requiring both functions. For example, the inventive device could be configured to generate a source signal and direct the source signal to an area of interest but a second device or transducer assembly could be used to receive the reflected signal. Accordingly, a separate device could be used to generate the source signal with the inventive device being used to receive the reflected signal.

The tip **304** may be comprised of materials such as a dimethyl pentene, a methylpentene copolymer (plastic-TPX), aluminum, carbon aerogel, polycarbonate (e.g., Lexan), polystyrene, or etc., any standard material used for ultrasound applications.

As illustrated in Fig. 6A, although the front surface **244** of the tip **302** is illustrated as being hemispherical, the tip **304** may have other profiles as well. For example, it is desirable that the tip **304** produce a certain amount of divergence of the signal being passed therethrough. However, depending on a variety of factors (e.g., material, frequency of the signal, etc.) a tip **304** may encounter excessive divergence which is destructive to the outgoing signal. Accordingly, it may be desirable to produce a tip **304** as illustrated in Fig. 6B in which a front surface **344** of the tip **304** is substantially flat. The degree of flatness of the tip **304** will often depend upon experimentation to reduce the amount of destructive reflections, thus minimizing excessive divergence due to differences in speed of sound in tip versus tissue. For example, when using a tip that is conducive to an ultrasound signal (e.g., TPX) a rounded tip can be used since there is not excessive divergence of the source signal. Use of a material that is not as conducive to ultrasound requires a flatter tip due to the resulting divergence of the source signal. Fig. 6C illustrates another variation of a tip **304** having a rounded front surface **344** but with no projections on the sides of the tip **304**. Fig. 6D illustrates a tip **304** with a concave front surface **344**.

In any case, the tip will be configured to avoid sharp edges that may cause any unintended damage to tissue while the device is being used to determine the presence or absence of a blood vessel. In such a case, for example, the tip may be designed such that it doesn't have sharp edges, or any sharp edges may be covered by other parts of the device (e.g., the elongate member, an outer sheath, etc.)

Commonly assigned patent publication nos. US20020128647A1; US20020138074A1; US20030130657A1, and US20050107783A1; disclose additional variations of transducer assemblies and modes of securing such assemblies to the device.

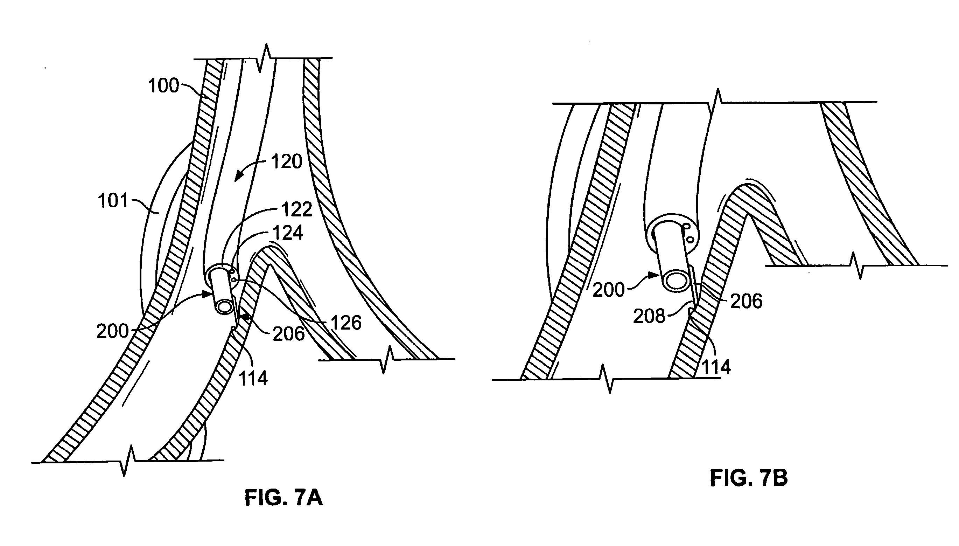

Figs. 7A-7E illustrates an example of use of the devices described herein. Although the figures show a device variation that is not in accordance with the present invention, it is contemplated that the device of the present invention may be substituted. In the illustrated example, the device creates an extra-anatomic passage in the airway wall tissue within a lung. However, it is understood that the device may be used in any part of the body and for any application. For example, variations of the device may be used during a biopsy procedure to scan for blood vessels, and remove a biopsy sample within the tissue piercing member.

Fig. 7A illustrates an access device **120** advanced into the airways **100** of a lung. The access device **120** may be a bronchoscope, endoscope, endotracheal tube with or without vision capability, or any type of delivery device. The access device **120** will have at least one lumen or working channel **122**. In the illustrated version, access device **120** includes a light **124** and vision **126** capabilities. In one example, the practitioner uses the access device **120** to locate an approximate site **114** for creation of a collateral channel. For example, location of the site may be accomplished visually, or with additional equipment such as a CT scan to locate areas for treatment. In cases where the access device **120** is a bronchoscope or similar device, the access device **120** is equipped so that the surgeon may observe the site for creation of the collateral channel. In some cases it may be desirable for non-invasive imaging of the procedure. In such cases, the access device **120** as well as the other devices discussed herein, may be configured for detection by the particular non-invasive imaging technique such as fluoroscopy, "real-time" computed tomography scanning, or other technique being used.

Fig. 7A also illustrates advancement of the device 200 through the channel **122** of the access device **120** towards the target site **114**. The medical practitioner then uses the sensing assembly **206** to inspect the target site **114** to determine whether a blood vessel **101** is adjacent to the site. If a blood vessel is detected at or near the site **114**, then another target site may be selected.

Fig. 7B shows a magnified view of the sensing assembly **206** of the device 200 being pressed against tissue at the target site **114**. Accordingly, variations of the sensing assembly **206** require sufficient stiffness so that the tissue may be adequately probed. As described above, the system **150** provides the medical practitioner with audio or visual signals so that the practitioner can determine whether it is sufficiently safe to make an opening in the tissue.

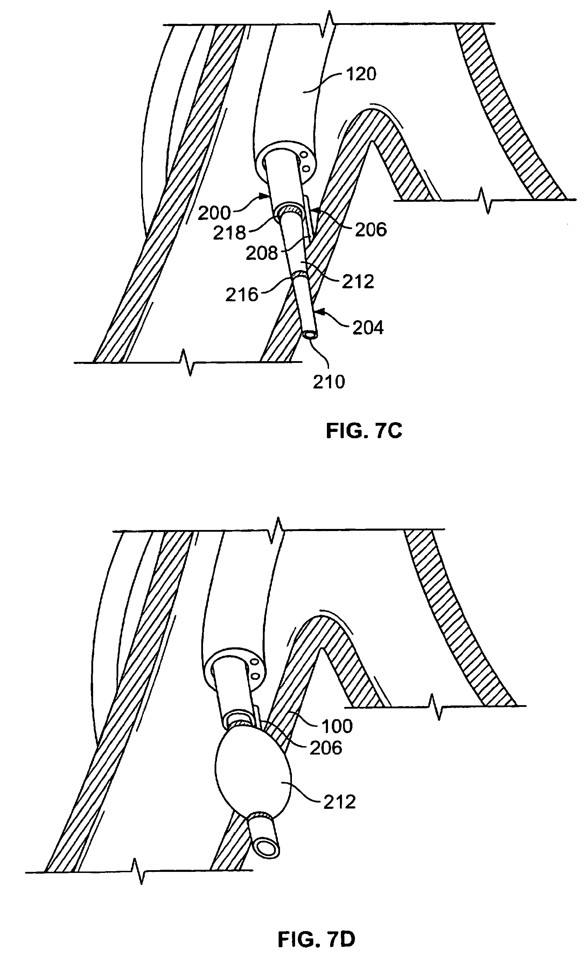

Fig. 7C illustrates the device **200** after the medical practitioner adequately determines that no blood vessel **101** is adjacent to the site 114. As show, the practioner advances the dilation member **204** into the tissue. Alternatively the sensing assembly may be used to create an opening in tissue. However, separating the tissue piercing function from the sensing function may offer an added safety feature by preventing inadvertent puncturing of tissue. The illustration also shows another useful feature of the device **200** as the sensing assembly **204** and sensing tip **208** can remain in contact with the tissue as the distal tip **208** of the dilation member **204** advances through tissue. As noted above, this feature is useful in environments where the target tissue moves.

Fig. 7C also illustrates the use of distal and proximal markers **216**, **218** on the device **200**. Although the markers **216**, **218** are shown to be adjacent to the expandable member **212**, the markers **216**, **218** may be placed anywhere along the device. However, placement of the markers **216**, **218** adjacent to the expandable member **212** permits visualization of the markers **216**, **218** so that the medical practitioner can minimize the chance that the proximal end of the expandable member **212** expands within the catheter member **202** or access device **120**.

Fig. 7D illustrates expansion of the expandable member **212**. As noted herein, the expandable member **212** may be a non-distensible balloon when the device is used in the lungs. However, variations of the device include the use of non-distensible balloons as well. Fig. 7D also illustrates deformation of the airway **100** wall upon expansion of the member **212**. Accordingly, the sensing assembly **206** may or may not be displaced during this process. Furthermore, as noted above, variations of the device may include sensors or transducers within the balloon. In such cases, the balloon's fluid couples the internal sensor/transducer to the surrounding tissue to perform an additional scan prior to placing any implant within the dilated opening.

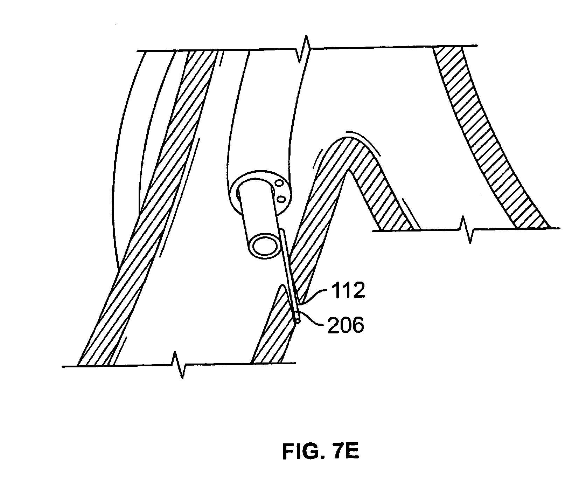

Fig. 7E illustrates an optional step that can take place after the dilation member **204** creates an opening **112** but before or after dilation of the opening **112**. In this device, the medical practitioner inserts the tip **208** of the sensing assembly **206** into the opening **112** to scan the area within or behind the airway wall. Accordingly, this feature is available when the size of the sensing tip **208** is less than a size of the tissue piercing member.

After dilation of the passage, the device may be removed. Alternatively, the expanded passage may be filled with fluid for additional scanning via the transducer assembly.

In one example of the procedure for placing implants in the airways to decompress hyper-inflated lungs, the medical practitioner selects a target location (usually via a CT scan) then confirms that the sensing assembly is function properly. This confirmation may be performed by placing the probe against a known region of tissue having a blood vessel. Next, the practitioner may scans the target site without knowing where a blood vessel is located. If the practitioner identifies a target site that appears to be free of blood vessels, the practioner may scan the areas around the target site to determine a vessel free region. Then, after selecting a site in the center of the region, the practioner creates the opening with a dilation member and subsequently dilates the opening. Some practitioners may scan in the opening prior to dilation and/or after dilation. Once the practitioner is satisfied that area is free of a blood vessel, the practitioner then inserts the implant.

A further variation of the invention may include configuring the transducer assembly and/or controller to have different levels of sensitivity. For example, a first level of sensitivity may be used to scan the surface of tissue. Then, after creation of the opening, the second level of sensitivity may be triggered. Such a feature acknowledges that scanning of tissue on, for example, the airway wall may require a different sensitivity than when scanning tissue within the parenchyma of the lung.

It should be noted that the invention includes kits containing the inventive device with any one or more of the following components, a Doppler ultrasound controller, a conduit as described in one or more of the applications listed above, and a bronchoscope/endoscope.

In the above explanation of Figs., similar numerals may represent similar features for the different variations of the invention.

The invention herein is described by examples and a desired way of practicing the invention is described. However, the invention as claimed herein is not limited to that specific description in any manner. Equivalence to the description as hereinafter claimed is considered to be within the scope of protection of this patent.

The devices of the present invention are configured to locate a target site for creation of a collateral channel in the tissue and to create an opening in tissue. As discussed above, a benefit of this combination feature is that a single device is able to select a target location and then create an opening without having been moved. Although the device is discussed as being primarily used in the lungs, the device is not limited as such and it is contemplated that the invention has utility in other areas as well, specifically in applications in which blood vessels or other structures must be avoided while cutting or removing tissue (one such example is tumor removal).

The above illustrations are examples of the invention described herein. It is contemplated that combinations of aspects of specific embodiments/variations or combinations of the specific embodiments/variations themselves are within the scope of this disclosure.

## Claims

1. A medical device (200) for sensing blood vessels in tissue and creating passages in the tissue, the device comprising:
a catheter member (202) having a near end, a far end, and a lumen extending therethrough;
a dilation member (204) within the lumen and having a shaft, the shaft having a distal tip (210) and a non-distensible expandable member (212) adjacent to the distal tip, the dilation member being slidably located within the catheter member; and
a sensing assembly (206) located at the far end of the catheter member; and
wherein the non-distensible expandable member is moveably located within the lumen independent of the sensing assembly, and the sensing assembly extends through the shaft of the dilation member, such that when the sensing assembly contacts tissue the dilation member may be advanced out of the catheter member and over the sensing member and into the tissue without removing the sensing member from the tissue.

2. The medical device of claim 1, wherein the sensing assembly (206) comprises a transducer assembly.

3. The medical device of claim 2, wherein the transducer assembly further includes a blunted tip.

4. The medical device of claim 2, wherein the transducer assembly comprises a Doppler ultrasound transducer assembly.

5. The medical device of claim 1, wherein the sensing assembly is adapted to penetrate soft tissue.

6. The medical device of claim 1, wherein a segment (211) of the sensing assembly (206) extends from the far end of the catheter member (202) and wherein the segment optionally
extends a distance from the far end of the catheter so that an end of the segment may contact tissue while a far end of the catheter does not contact tissue;
a diameter of the segment is less than a diameter of the distal tip, such that the segment may advance into an opening in tissue created by the distal tip; or
wherein the segment has sufficient stiffness to provide force against tissue when pressed thereon and optionally a portion of the sensing assembly proximal to the segment has a low column strength.

7. The medical device of claim 1, wherein the distal tip of the dilation member (204) comprises a cannula, and wherein the non-distensible expandable member (212) is located about the cannula.

8. The medical device of claim 1, wherein the non-distensible expandable member (212) is adapted to expand in a radial direction from the shaft.

9. The medical device of claim 1, further comprising a tissue piercing member on the distal tip of the shaft, wherein the tissue piercing member is able to penetrate soft tissue.

10. The medical device of claim 9, wherein a cross sectional size of the sensing assembly is less than a cross-sectional size of the distal tip such that the sensing assembly can be inserted into the opening created by the tissue piercing member.

11. The medical device of claim 1, wherein the shaft of the dilation member (204) comprises a polymeric tube and wherein the shaft optionally comprises a reinforced polymeric tube or the distal tip comprises a beveled tip.

12. The medical device of claim 1, further comprising a handle (196) at a near end of the catheter member (202), the handle having a first moveable actuator coupled to the dilation member.

13. The medical device of claim 1, wherein the dilation member (204) further comprises a support member extending therethrough, wherein the support member has sufficient column strength to drive the distal tip (210) into tissue.

14. The medical device of claim 1, wherein the dilation member (204) further comprises either:
a first marker (216) located at a distal end of the non-distensible expandable member (212), or a second marker (218) located at a proximal end of the non-distensible expandable member; and
wherein the first and second markers are either a visible or a radiopaque markers.

15. A system for sensing blood vessels in tissue and creating passages in the tissue, the system comprising:
the device (200) of claim 1;
a Doppler control unit (190) adapted to be coupled to the near end of the catheter; and
a fluid source (192) adapted to be coupled to the dilation member (204); and
wherein the sensing assembly (206) comprises a Doppler ultrasound transducer assembly.

## Patentansprüche

1. Medizinische Vorrichtung (200) zum Sensieren von Blutgefäßen in Gewebe und Erzeugen von Durchgängen in dem Gewebe, wobei die Vorrichtung umfasst:
ein Katheterteil (202) mit einem nahen Ende, einem fernen Ende und einem sich dadurch erstreckenden Lumen;
ein Erweiterungsteil (204) innerhalb des Lumens und mit einem Schaft, wobei der Schaft eine distale Spitze (210) und ein nicht-dehnbares expandierbares Teil (212) angrenzend an die distale Spitze hat, wobei sich das Erweiterungsteil verrutschbar innerhalb des Katheterteils befindet; und
eine Sensieranordnung (206), die sich an dem fernen Ende des Katheterteils befindet; und
wobei sich das nicht-dehnbare expandierbare Teil unabhängig von der Sensieranordnung beweglich innerhalb des Lumens befindet und sich die Sensieranordnung durch den Schaft des Erweiterungsteils hindurch erstreckt, sodass, wenn die Sensieranordnung Gewebe kontaktiert, das Erweiterungsteil aus dem Katheterteil hervorgeschoben und über das Sensierteil und in das Gewebe hineingeschoben werden kann, ohne das Sensierteil von dem Gewebe zu entfernen.

2. Medizinische Vorrichtung nach Anspruch 1, wobei die Sensieranordnung (206) eine Transducer-Anordnung umfasst.

3. Medizinische Vorrichtung nach Anspruch 2, wobei die Transducer-Anordnung weiterhin eine stumpfe Spitze beinhaltet.

4. Medizinische Vorrichtung nach Anspruch 2, wobei die Transducer-Anordnung eine Doppler-Ultraschall-Transducer-Anordnung umfasst.

5. Medizinische Vorrichtung nach Anspruch 1, wobei die Sensieranordnung eingerichtet ist, um in Weichgewebe einzudringen.

6. Medizinische Vorrichtung nach Anspruch 1, wobei sich ein Segment (211) der Sensieranordnung (206) von dem fernen Ende des Katheterteils (202) erstreckt und wobei sich das Segment optional über eine Entfernung von dem fernen Ende des Katheters erstreckt, sodass ein Ende des Segments Gewebe kontaktieren kann, während ein fernes Ende des Katheters kein Gewebe kontaktiert;
wobei ein Durchmesser des Segments geringer als ein Durchmesser der distalen Spitze ist, sodass das Segment in eine Öffnung in Gewebe vorgeschoben werden kann, die durch die distale Spitze erzeugt wird; oder
wobei das Segment ausreichende Steifigkeit hat, um Kraft gegen Gewebe bereitzustellen, wenn darauf gedrückt wird, und wobei optional ein Abschnitt der Sensieranordnung proximal des Segments eine geringe Stützstärke hat.

7. Medizinische Vorrichtung nach Anspruch 1, wobei die distale Spitze des Erweiterungsteils (204) eine Kanüle umfasst, und wobei sich das nicht-dehnbare expandierbare Teil (212) um die Kanüle herum befindet.

8. Medizinische Vorrichtung nach Anspruch 1, wobei das nicht-dehnbare expandierbare Teil (212) eingerichtet ist, um in einer radialen Richtung von dem Schaft zu expandieren.

9. Medizinische Vorrichtung nach Anspruch 1, weiterhin ein Gewebestichteil auf der distalen Spitze des Schafts umfassend, wobei das Gewebestichteil in Weichgewebe eindringen kann.

10. Medizinische Vorrichtung nach Anspruch 9, wobei eine Querschnittsgröße der Sensieranordnung geringer als eine Querschnittsgröße der distalen Spitze ist, sodass die Sensieranordnung in die Öffnung, die von dem Gewebestichteil erzeugt wird, eingeführt werden kann.

11. Medizinische Vorrichtung nach Anspruch 1, wobei der Schaft des Erweiterungsteils (204) ein Polymerrohr umfasst und wobei der Schaft optional ein verstärktes Polymerrohr umfasst oder die distale Spitze eine abgeschrägte Spitze umfasst.

12. Medizinische Vorrichtung nach Anspruch 1, weiterhin einen Griff (196) an einem nahen Ende des Katheterteils (202) umfassend, wobei der Griff eine erste bewegliche Betätigungseinheit hat, die mit dem Erweiterungsteil gekoppelt ist.

13. Medizinische Vorrichtung nach Anspruch 1, wobei das Erweiterungsteil (204) weiterhin ein Stützteil umfasst, das sich durch dieses erstreckt, wobei das Stützteil eine ausreichende Stützstärke hat, um die distale Spitze (210) in Gewebe zu treiben.

14. Medizinische Vorrichtung nach Anspruch 1, wobei das Erweiterungsteil (204) weiterhin entweder umfasst:
einen ersten Marker (216), der sich an einem distalen Ende des nicht-dehnbaren expandierbaren Teils (212) befindet, oder einen zweiten Marker (218), der sich an einem proximalen Ende des nicht-dehnbaren expandierbaren Teils befindet; und
wobei der erste und zweite Marker entweder sichtbar sind oder röntgenfähige Marker sind.

15. System zum Sensieren von Blutgefäßen in Gewebe und zum Erzeugen von Durchgängen in dem Gewebe, wobei das System umfasst:
die Vorrichtung (200) nach Anspruch 1;
eine Doppler-Steuereinheit (190), die eingerichtet ist, um mit dem nahen Ende des Katheters gekoppelt zu werden; und
eine Flüssigkeitsquelle (192), die eingerichtet ist, um mit dem Erweiterungsteil (204) gekoppelt zu werden; und
wobei die Sensieranordnung (206) eine Doppler-Ultraschall-Transducer-Anordnung umfasst.

## Revendications

1. Dispositif médical (200) permettant de détecter les vaisseaux sanguins dans un tissu et de créer des passages dans le tissu, le dispositif comprenant :
un élément de cathéter (202) ayant une extrémité proche, une extrémité éloignée et une lumière s'étendant à travers celui-ci ;
un élément de dilatation (204) dans la lumière, comportant un axe, l'axe ayant un bout distal (210) et un élément extensible mais non dilatable (212) adjacent au bout distal, l'élément de dilatation étant situé de façon glissante à l'intérieur de l'élément de cathéter ; et
un ensemble de détection (206) situé à l'extrémité éloignée de l'élément de cathéter ; et
dans lequel l'élément extensible non dilatable est placé de façon mobile à l'intérieur de la lumière, indépendamment de l'ensemble de détection, et l'ensemble de détection s'étend dans l'axe de l'élément de dilatation, de sorte que lorsque l'ensemble de détection entre en contact avec un tissu, l'élément de dilatation peut être poussé hors de l'élément de cathéter pour passer par dessus l'élément de détection et pénétrer dans le tissu sans retirer l'élément de détection du tissu.

2. Dispositif médical selon la revendication 1, dans lequel l'ensemble de détection (206) comprend un ensemble transducteur.

3. Dispositif médical selon la revendication 2, dans lequel l'ensemble transducteur comprend en outre un bout émoussé.

4. Dispositif médical selon la revendication 2, dans lequel l'ensemble transducteur comprend un ensemble transducteur à ultrasons Doppler.

5. Dispositif médical selon la revendication 1, dans lequel l'ensemble de détection est adapté pour pénétrer dans un tissu mou.

6. Dispositif médical selon la revendication 1, dans lequel un segment (211) de l'ensemble de détection (206) s'étend depuis l'extrémité éloignée de l'élément de cathéter (202) et dans lequel en option :
le segment s'étend à une distance de l'extrémité éloignée du cathéter de telle manière qu'une extrémité du segment peut entrer en contact avec le tissu tandis qu'une extrémité éloignée du cathéter ne touche pas le tissu ;
un diamètre du segment est inférieur à un diamètre du bout distal, de sorte que le segment peut avancer dans une ouverture créée dans le tissu par le bout distal ; ou
dans lequel le segment a une rigidité suffisante pour fournir une force contre le tissu quand il est pressé sur celui-ci et en option une partie de l'ensemble de détection proche du segment a une faible résistance en colonne.

7. Dispositif médical selon la revendication 1, dans lequel le bout distal de l'élément de dilatation (204) comprend une canule, et dans lequel l'élément extensible non dilatable (212) est placé autour de la canule.

8. Dispositif médical selon la revendication 1, dans lequel l'élément extensible non dilatable (212) est adapté pour réaliser une extension dans une direction radiale depuis l'axe.

9. Dispositif médical selon la revendication 1, comprenant en outre un élément perçant le tissu sur le bout distal de l'axe, dans lequel l'élément perçant le tissu est apte à pénétrer dans un tissu mou.

10. Dispositif médical selon la revendication 9, dans lequel une taille en section transversale de l'ensemble de détection est inférieure à une taille en section transversale du bout distal, de sorte que l'ensemble de détection peut être inséré dans l'ouverture créée par l'élément perçant le tissu.

11. Dispositif médical selon la revendication 1, dans lequel l'axe de l'élément de dilatation (204) comprend un tube en polymère et dans lequel l'axe comprend en option un tube en polymère renforcé ou le bout distal comprend un bout biseauté.

12. Dispositif médical selon la revendication 1, comprenant en outre une poignée (196) à une extrémité proche de l'élément de cathéter (202), la poignée ayant un premier actionneur mobile couplé à l'élément de dilatation.

13. Dispositif médical selon la revendication 1, dans lequel l'élément de dilatation (204) comprend en outre un élément de support s'étendant à travers celui-ci, dans lequel l'élément de support a une résistance en colonne suffisante pour enfoncer le bout distal (210) dans un tissu.

14. Dispositif médical selon la revendication 1, dans lequel l'élément de dilatation (204) comprend en outre :
soit un premier marqueur (216) situé à une extrémité distale de l'élément extensible non dilatable (212), soit un deuxième marqueur (218) situé à une extrémité proximale de l'élément extensible non dilatable ; et
dans lequel les premier et deuxième marqueurs sont soit des marqueurs visibles soit des marqueurs radio-opaques.

15. Système permettant de détecter les vaisseaux sanguins dans un tissu et de créer des passages dans le tissu, le système comprenant :
le dispositif (200) de la revendication 1 ;
une unité de commande Doppler (190) adaptée pour être couplée à l'extrémité proche du cathéter ; et
une source de fluide (192) adaptée pour être couplée à l'élément de dilatation (204) ; et
dans lequel l'ensemble de détection (206) comprend un ensemble transducteur à ultrasons Doppler.
